# EUROPEAN PATENT SPECIFICATION

(11) **EP 2 227 216 B1**
(45) Date of publication and mention of the grant of the patent: **28.06.2017**
(21) Application number: 08859603.6
(22) Date of filing: 11.12.2008
(51) Int. Cl.: A61K 8/06, A61K 8/31, A61K 8/35, A61K 8/67, A61K 9/107, A61K 47/08, A61Q 19/00, A61K 8/55, A61K 8/92, A61K 8/97

(54) **SKIN EXTERNAL PREPARATION AND METHOD OF PRODUCING THE SAME**
TOPISCHES HAUTPRÄPARAT UND HERSTELLUNGSVERFAHREN DAFÜR
PRÉPARATION EXTERNE POUR LA PEAU ET SON PROCÉDÉ DE FABRICATION

(30) Priority: 12.12.2007 JP 2007321395
(43) Date of publication of application: 15.09.2010
(73) Proprietor: FUJIFILM Corporation, Tokyo 106-8620 (JP)
(72) Inventor: NAKAMURA, Yoshisada, Ashigarakami-gun Kanagawa 258-8577 (JP)
(74) Representative: Klunker IP Patentanwälte PartG mbB
(86) International application number: PCT/JP2008/073021
(87) International publication number: WO 2009/075383

(56) References cited:
- EP-A1- 1 264 595
- EP-A1- 1 616 552
- JP-A- 2 174 927
- JP-A- 2002 338 433
- JP-A- 2004 285 026
- JP-A- 2006 008 690
- JP-A- 2007 269 749
- JP-A- 2007 270 073
- US-A- 4 824 877
- US-A1- 2006 204 469

## Description

### Technical Field

The invention relates to a skin external preparation and a method of producing the same, and particularly relates to a skin external preparation of an oil-in-water emulsion composition containing a functional oil component and a method of producing the same.

### Background Art

Various emulsion compositions capable of effectively demonstrating functions of various functional oil components have been developed with expectation regarding the efficacies thereof. For example, carotenoids are naturally-occurring yellow to red terpenoid colorants, and are known to have strong antioxidant effects. In particular, astaxanthins (including astaxanthin, esters thereof, and the like) which are a kind of carotenoid are known to have functions such as an antioxidant effect, an antiinflammatory effect (e.g., Japanese Patent Application Laid-Open (JP-A) No. 02-049091), an anti-skin aging effect (e.g., JP-A No. 05-155736) or an effect of preventing the formation of spots or wrinkles (e.g., JP-A No. 2005-047860). Therefore, various foodstuffs, cosmetics, pharmaceuticals and the like, containing astaxanthins have been developed.

In JP-A No. 05-155736, emulsification is performed using an astaxanthin in a solvent extract solution from krill as one of the oil phase components to thereby obtain emollient cream or the like.

In JP-A No. 2003-055188, a water-soluble form of astaxanthin is used for face lotion or the like, and an oil form of astaxanthin is used for cream or the like. These oil-soluble ingredients are mixed and emulsified with other oil phase components and water phase components to produce skin external preparations in target forms. Emulsion compositions are also disclosed in US 4,824,877, which teaches a polydiorganosiloxane emulsion; US 2006/204469, which teaches a stable emulsion containing both nanoemulsion and macroemulsion semisolid dispersions; and EP1264595, which teaches the use of tocopherol for stabilising lecithin nanmemulsions.

However, in order to further improve the functions of such functional oil components to be more effective, the above-described measures have not been sufficient.

### Disclosure of Invention

An object of the invention is to provide a skin external preparation capable of effectively exhibiting the functions of a functional oil component, and a method of producing the same.

The skin external preparation of the invention includes an oil-in-water emulsion composition. The emulsion composition contains first oil droplet particles having a minimum particle diameter of 200 nm or more and second oil droplet particles having a volume average particle diameter determined by a dynamic light scattering method of 70 nm or less, the first oil droplet particles contain a first functional oil component, and the second oil droplet particles contain a second functional oil component which may be the same as or different from the first functional oil component wherein the minimum particle diameter refers to a value obtained by subtracting a value that is three times the standard deviation of a volume-particle size distribution from a volume average particle diameter, the volume-particle size distribution and the volume average particle diameter being determined by a dynamic light scattering method, wherein each of the first functional oil component and the second functional oil component is independently at least one selected from the group consisting of an oil and a fat, a hydrocarbon, a wax, an ester, a fatty acid, a higher alcohol, a polymer, an oil-soluble colorant and an oil-soluble protein, and wherein at least one of the first functional oil component or the second functional oil component comprises at least one of astaxanthin or an ester of astaxanthin

At least one of (i.e., one of, or both of) the first functional oil component or the second functional oil component may include a Haematococcus alga extract. Furthermore, the content of the functional oil components may be from 0.2 mass% to 10 mass% with respect to the total amount of the emulsion composition.

In the skin external preparation, the amount of the first oil droplet particles may be from 0.005 mass% to 60 mass% with respect to the total amount of the emulsion composition.

Moreover, in the skin external preparation, the amount of the second oil droplet particles may be from 0.005 mass% to 60 mass% with respect to the total amount of the emulsion composition.

Furthermore, in the skin external preparation, it is preferable that the blending ratio of the first oil droplet particles to the second oil droplet particles in the composition be from 50,000:1 to 1:50,000 by mass.

In the skin external preparation, the total blending amount of oil phase components in the oil-in-water emulsion composition may be from 0.01 mass% to 60 mass% with respect to the total amount of the emulsion composition.

The skin external preparation may further include a water-soluble compound selected from the group consisting of a whitening agent, an anti-wrinkle agent, an antioxidant, a skin-roughness improving agent, a protective agent for stratum corneum, an agent for acne treatment and an astringent.

The method of producing the skin external preparation of the invention is a method of producing the above-mentioned skin external preparation, and includes mixing a first emulsion containing the first oil droplet particles having a minimum particle diameter of 200 nm or more and a second emulsion containing the second oil droplet particles having an average particle diameter of 70 nm or less.

Here, in the method of producing the skin external preparation, it is preferable that the mixing ratio of the first emulsion and the second emulsion be from 50,000:1 to 1:50,000 by mass.

Furthermore, in the method of producing the skin external preparation, it is preferable that the content of the first functional oil component in the first emulsion is from 5 mass% to 50 mass%, and the content of the second functional oil component in the second emulsion is from 5 mass% to 35mass%.

According to the invention, a skin external preparation capable of effectively demonstrating the functions of a functional oil component, and a method of producing the same are provided.

### Best Mode for Carrying Out the Invention

The skin external preparation of the invention is an oil-in-water emulsion composition containing a functional oil component. In the skin external preparation, the emulsion composition contains at least (i) first oil droplet particles having a minimum particle diameter of 200 nm or more and (ii) second oil droplet particles having a volume average particle diameter determined by a dynamic light scattering method of 70 nm or less, the first oil droplet particles contain a functional oil component, and the second oil droplet particles also contain a functional oil component. The functional oil component contained in the first oil droplet particles and the functional component contained in the second oil droplet particles may be the same as or different from each other and wherein the minimum particle diameter refers to a value obtained by subtracting a value that is three times the standard deviation of a volume-particle size distribution from a volume average particle diameter, the volume-particle size distribution and the volume average particle diameter being determined by a dynamic light scattering method, wherein each of the first functional oil component and the second functional oil component is independently at least one selected from the group consisting of an oil and a fat, a hydrocarbon, a wax, an ester, a fatty acid, a higher alcohol, a polymer, an oil-soluble colorant and an oil-soluble protein, and wherein at least one of the first functional oil component or the second functional oil component comprises at least one of astaxanthin or an ester of astaxanthin.

As described above, the emulsion composition of the invention contains the first oil droplet particles and the second oil droplet particles having different particle diameters from those of the first oil droplet particles. Therefore, when the skin external preparation is applied to the skin, the first oil droplet particles having a minimum particle diameter of 200 nm or more remain on the stratum corneum since the first oil droplet particles are larger than the cell-to-cell distance in the stratum corneum. On the other hand, the second oil droplet particles having avolume average particle diameter determined by a dynamic light scattering method of 70 nm or less can penetrate into the stratum corneum.

Thus, the functional oil component can be effectively arranged on the outside and the inside of the stratum corneum according to the arrangement of oil droplet particles of each kind (first or second oil droplet particles), and the functions of the functional oil component(s) can be effectively exhibited.

The first oil droplet particles contained in the emulsion composition of the invention are oil-in-water particles, and have a minimum particle diameter of 200 nm or more. When the minimum particle diameter is 200 nm or more, the oil droplet particles do not pass through the stratum corneum, and can thus be arranged on the surface of the stratum corneum. In consideration of sense of use, the minimum particle diameter of the first oil droplet particles is preferably 400 nm or more, and more preferably 2,000 nm or more.

The first oil droplet particles can be easily obtained by ordinary methods of producing an oil-in-water emulsion. In the invention, an oil-in-water emulsion containing the first oil droplet particles is referred to as a first emulsion.

The minimum particle diameter as used herein refers to a value obtained by subtracting a value that is three times the standard deviation of a volume-particle size distribution from a volume average particle diameter determined by particle diameter measurement, and can be determined by the particle size distribution measuring method described below.

The second oil droplet particles contained in the emulsion composition of the invention are oil-in-water particles similar to the first oil droplet particles, and have avolume average particle diameter determined by a dynamic light scattering method of 70 nm or less. When the average particle diameter is 70 nm or less, the oil droplet particles can penetrate into the stratum corneum. In consideration of penetration ability, the volume average particle diameter of the second oil droplet particles as determined by a dynamic light scattering method is preferably 50 nm or less, and more preferably 20 nm or less.

The volume average particle diameter in the invention can be measured with commercially available measurement devices using dynamic light scattering such as a NANOTRAC UPA (trade name, Nikkiso Co., Ltd.), a dynamic light scattering particle size distribution measuring device LB-550 (trade name, Horiba, Ltd.) and a fiber-optics particle size analyzer FPAR-1000 (trade name, Otsuka Electronics Co., Ltd.). The measurement temperature may be a temperature generally used for measuring the particle diameter, and is preferably 20°C.

The particle diameter in the invention refers to a value measured at 20°C with a dynamic light scattering particle size distribution measuring device.

The second oil droplet particles can be easily obtained by an oil-in-water emulsion producing method described below. In the invention, an oil-in-water emulsion containing the second oil droplet particles is referred to as a second emulsion.

The emulsion composition of the invention contains the first oil droplet particles and the second oil droplet particles. In order for the first oil droplet particles to have functions of protecting the skin on the skin surface and assisting a skin barrier function, the emulsion composition of the invention may contain the first oil droplet particles at a content of from 0.005 mass% to 60 mass%, preferably from 0.1 mass% to 35 mass%, and more preferably from 5 mass% to 25 mass%.

On the other hand, in order for the second oil droplet particles to have functions of protecting the skin cells and extracellular components in the skin and assisting a skin barrier function, the emulsion composition may contain the second oil droplet particles at a content of from 0.005 mass% to 60 mass%, preferably from 0.01 mass% to 15 mass%, and more preferably from 0.5 mass% to 5 mass%.

The blending ratio of the first oil droplet particles to the second oil droplet particles changes depending on which function is to be emphasized-the protective function of the skin surface or inside the skin or the barrier function-, and may be selected from the range of from 50,000:1 to 1:50,000 by mass. In particular, in consideration of a required amount of ingredients needed for the protection of the skin surface and the assistance of the barrier function, the blending ratio of the first oil droplet particles to the second oil droplet particles is preferably from 100:1 to 1:10, and more preferably from 10:1 to 1:2, by mass.

In the emulsion composition of the invention, the first oil droplet particles contain a functional oil component and the second oil droplet particles also contains a functional oil component. The following description of "functional oil component" applies both of the functional oil component in the first oil droplet particles and the functional oil component in the second oil droplet particles.

### (a) Functional oil component

As the functional oil component used in the invention, an oil-soluble antioxidant component is preferable in consideration of effects when applied to the skin. Examples of the oil-soluble antioxidant component include carotenoids, vitamin Es (such as tocopherol or tocotrienol), ubiquinones, and ω-3 oils and fats (such as oils and fats containing, for example, EPA, DHA, or linolenic acid).

Preferred examples of carotenoids in the invention include carotenoids containing natural colorants, and examples of the natural colorants include colorants of yellow to red terpenoids, which may be derived from plants, algae or bacteria.

Examples of the carotenoids include hydrocarbons (carotenes) and their oxidized alcohol derivatives (xanthophylls).

Examples thereof include actinioerythrol, astaxanthin, bixin, canthaxanthin, capsanthin, capsorbin, β-8'-apocarotenal (apocarotenal), β-12'-apocarotenal, α-carotene, β-carotene, "carotene" (a mixture of α- and β-carotenes), γ-carotene, β-cryptoxanthin, echinenone, lutein, lycopene, violerythrin, zeaxanthin, fucoxanthin and esters of compounds having a hydroxyl or carboxyl group selected from the above.

Carotenoids used in the invention are preferably oily at ordinary temperature inconsideration of making the emulsion particle diameter smaller. A particularly preferable example of the carotenoids may include at least one selected from astaxanthin and astaxanthin derivatives (hereinafter generically referred to as "astaxanthins") such as esters of astaxanthin, which have antioxidant effects, antiinflammatory effects, skin antiaging effects, whitening ability and the like and which are known as yellow to red colorants.

Astaxanthin is a red colorant having an absorption maximum at 476 nm (in ethanol), 468 nm (in hexane), and belongs to xanthophylls - one kind of carotenoid. The chemical structure of astaxanthin is 3,3'-dihydroxy-β,β-carotene-4,4'-dione (C₄₀H₅₂O₄ with a molecular weight of 596.82).

The astaxanthin and/or its ester (astaxanthins) may be used in the form of an astaxanthin-containing oil separated and extracted from natural products containing astaxanthin and/or an astaxanthin ester. Examples of the astaxanthin-containing oil include extracts, such as extracts extracted from a culture of red yeast Phaffia, green alga Haematococcus, marine bacteria or the like and extracts from Antarctic krill or the like.

Astaxanthins that may be used in the invention include the above extracted products (extracts), products obtained by appropriately purifying the extracts as necessary, and synthetic products. As the astaxanthins, products extracted from Haematococcus algae (hereinafter sometimes referred to as "Haematococcus alga extract") are particularly prefable in consideration of quality and productivity.

In the invention, commercially available Haematococcus alga extracts may be used, and examples thereof include: ASTOTS-S, ASTOTS-2.5 O, ASTOTS-5 O and ASTOTS-10 O (trade names, manufactured by Takedashiki Co., Ltd.); AstaREAL oil 50F and AstaREAL oil 5F (trade names, manufactured by Fuji Chemical Industry Co., Ltd.); and BioAstin SCE7 (trade name, manufactured by Toyo Koso Kagaku Co., Ltd).

In the invention, the content of astaxanthins as a colorant pure component in Haematococcus alga extract is preferably from 0.001 mass% to 50 mass%, and more preferably from 0.01 mass% to 25 mass%, in consideration of extraction cost.

In addition to carotenoid colorants, preferable examples of the functional oil component include ubiquinones, particularly coenzyme Q10.

Preferable examples of the functional oil component in the invention further include ω(omega)-3 oils and fats of unsaturated fatty acids having a double bond at ω-3 position. Examples of the ω(omega)-3 oils and fats include linolenic acid, eicosapentaenoic acid (EPA), docosahexaenoic acid (DHA), and fish oils containing those.

Other examples of a compound that may be used as the functional oil component include liquid oils (fatty oils), which are liquid at ordinary temperature, and fats, which are solid at ordinary temperature.

Examples of liquid oils include olive oil, camellia oil, macadamia nut oil, castor oil, avocado oil, evening primrose oil, turtle oil, corn oil, mink oil, rapeseed oil, egg yolk oil, sesame oil, persic oil, wheat germ oil, sasanqua oil, linseed oil, safflower oil, cotton seed oil, perilla oil, soybean oil, earthnut oil, tea seed oil, kaya oil, rice bran oil, china wood oil, tung oil, hohoba oil, germ oil, triglycerin, glycerin trioctanoate, glycerin triisopalmitate, salad oil, safflower oil, palm oil, coconut oil, peanut oil, almond oil, hazelnut oil, walnut oil, grape seed oil, squalene, and squalane.

Examples of solid fats include beef tallow, hydrogenated beef tallow, neet's-foot tallow, beef bone tallow, mink oil, egg yolk oil, lard, horse fat, mutton tallow, hydrogenated oil, cacao fat, coconut oil, hydrogenated coconut oil, palm oil, palm hydrogenated oil, Japan tallow, Japan tallow kernel oil and hydrogenated castor oil.

Other examples of the functional oil component include: hydrocarbons such as liquid paraffin, paraffin, Vaseline, ceresin or microcrystalline wax; waxes such as carnauba wax, candellia wax, jojoba oil, bees wax or lanolin; esters such as isopropyl myristate, 2-octyldedecyl myristate, cetyl 2-ethylhexanoate or diisostearyl malate; fatty acids such as palmitic acid, stearic acid, isostearic acid, linoleic acid or arachidonic acid; higher alcohols such as cetyl alcohol, stearyl alcohol, isostearyl alcohol or 2-octyldodecanol; silicone oils such as methyl polysiloxane or methylphenyl polysiloxane; polymers; oil-soluble colorants; oil-soluble proteins; and various plant-derived oils and animal-derived oils, which are mixtures of substances selected from the above substances.

As described above, each of the first emulsion and the second emulsion a functional oil component, which may be selected from the above. In consideration of the application of the emulsion composition, the emulsion particle diameter and the emulsion stability, the total amount of the functional oil components is preferably from 0.1 mass% to 50 mass%, more preferably from 0.5 mass% to 25 mass%, and still more preferably from 1 mass% to 10 mass% with respect to the total amount of the emulsion composition.

When the content of the functional oil components is 0.1 mass% or more, sufficient amount of an active component is contained, and the emulsion composition may be easily applied to foods and cosmetics. When the content of the oil components is 50 mass% or less, increase in the emulsion particle diameter and deterioration of the emulsion stability may be suppressed.

The content of the functional oil component in each of the first emulsion and second emulsion depends on the type of the functional oil component and the like. In general, in consideration of balance between sense of use and functions, the content of the functional oil component in the first emulsion is from 1 mass% to 85 mass%, and preferably from 5 mass% to 50 mass%, with respect to the total amount of the first emulsion. On the other hand, in consideration of functions, the content of the functional oil component in the second emulsion is from 1 mass% to 50 mass%, and preferably from 5 mass% to 35 mass%, with respect to the total amount of the second emulsion.

### (b) Radical scavenger

The emulsion composition preferably contains, as an oil component, a lipid-soluble radical scavenger (antioxidant) having a function of scavenging a radical.

In a preferable embodiment, from viewpoint of preventing oxidation of another oil component, the radical scavenger is used alone, or two or more thereof are used in combination.

Examples of a compound that may be used as a radical scavenger in the invention include compounds having a phenolic OH group, amine compounds such as phenylenediamine, oil-solubilized derivatives of ascorbic acid, and oil-solubilized derivatives of erythorbic acid.

The content of the radical scavenger in the emulsion composition is generally from 0.001 mass% to 20.0 mass%, preferably from 0.01 mass% to 10 mass%, and more preferably from 0.1 mass% to 5.0 mass%, with respect to the total amount of the emulsion composition.

In the invention, each of the first emulsion and the second emulsion includes a functional oil component. In view of this constitution, each of the first and the second emulsion may include a radical scavenger, or only one of the first or the second emulsion may include a radical scavenger. When either one of the first or the second emulsion includes a radical scavenger, the radical scavenger is preferably included in the first emulsion in view of exhibiting antioxidant effects of the functional oil component. When each of the first emulsion and the second emulsion includes a radical scavenger, the content of the radical scavenger can be suitably set according to the content of the functional oil component in each of the first emulsion and the second emulsion.

Examples of the compounds having a phenolic OH group include polyphenols (such as catechin), guaiacum, nordihydroguaiaretic acid (NDGA), gallic acid esters, BHT (butylhydroxytoluene), BHA (butylhydroxyanisol), vitamin Es and bisphenols. Examples of the gallic acid esters include propyl gallate, butyl gallate and octyl gallate.

Examples of the amine compounds include phenylenediamine, diphenyl-p-phenylenediamine and 4-amino-p-diphenylamine. Among these compounds, diphenyl-p-phenylenediamine and 4-amino-p-diphenylamine are more preferable.

Examples of the oil-solubilized derivatives of ascorbic acid and the oil-solubilized derivatives of erythorbic acid include L-ascorbyl stearate, L-ascorbyl tetraisopalmitate, L-ascorbyl palmitate, erythorbyl palmitate, and erythorbyl tetraisopalmitate.

Among the above compounds, vitamin Es are particularly preferably used in consideration of safety and their excellent antioxidant function.

Vitamin Es used in the invention are not particularly limited, and examples thereof include: a class of compounds including tocopherol and derivatives thereof; and another class of compounds including tocotrienol and derivatives thereof. It is possible to use one of these compounds or to use two or more of these compounds in combination. Furthermore, a compound selected from the class of compounds including tocopherol and derivatives thereof and a compound selected from the class of compounds including tocotrienol and derivatives thereof may be used in combination.

Examples of the class of compounds including tocopherol and derivatives thereof include dl-α-tocopherol, dl-β-tocopherol, dl-γ-tocopherol, dl-δ-tocopherol, acetic acid dl-α-tocopherol ester, nicotinic acid dl-α-tocopherol ester, linoleic acid dl-α-tocopherol ester and succinic acid dl-α-tocopherol ester. Of those, dl-α-tocopherol, dl-β-tocopherol, dl-γ-tocopherol, dl-δ-tocopherol and a mixture of two or more of these compounds (mix tocopherol) are more preferred. Preferable examples of the tocopherol derivatives include acetic acid esters of the compounds described above.

Examples of the class of compounds including tocotrienol and derivatives thereof include α-tocotrienol, β-tocotrienol, γ-tocotrienol and δ-tocotrienol. Preferable examples of the tocotrienol derivatives include acetic acid esters of the compounds described above. Tocotrienol is a tocopherol analogue contained in wheat, barley, rye, oat, rice bran, palm oil and the like. Tocotrienol contains three double bonds in a side chain of tocopherol, and has excellent antioxidant performance.

Among the above vitamin Es, the emulsion composition preferably contains at least one compound selected from the class of compounds including tocotrienol and derivatives thereof, in consideration of the antioxidant effect.

### (c) Emulsifier

The emulsion composition in the invention may contain at least one emulsifier selected from the group consisting of phospholipids and surfactants in order to obtain the first oil droplet particles and the second oil droplet particles.

### Phospholipid

In the present invention, the term "phospholipids" refers to a class of complex lipids. A phospholipid is an ester containing fatty acid, alcohol, phosphoric acid, and optionally a nitrogen compound, and has at least one phosphoric ester portion and at least one fatty acid ester portion. Examples of phospholipids include glycerophospholipids having glycerin as a basic skeleton and sphingophospholipids having sphingosine as a basic skeleton.

Specific examples of phospholipids that may be used in the present invention include phosphatidic acid, bisphosphatidic acid, lecithin (phosphatidylcholine), phosphatidylethanolamine, phosphatidyl methylethanolamine, phosphatidylserine, phosphatidylinositol, phosphatidylglycerol, diphosphatidylglycerol, and sphingomyelin. Examples of phospholipids further include lecithins containing such phospholipids and derived from, for example, plants such as soybean, corn, peanut, rapeseed, wheat, barley, rye, and oat, animals such as egg yolk and a cow; and microorganisms such as Escherichia coli. Lecithins and hydrogenated lecithins, which are mixtures of such substances as described above, are also usable. There is no limitation on the origins of the above phospholipids, but a purified phospholipid is preferable. In the present invention, the scope of glycerophospholipid includes a lysolecithin - a glycerophospholipid having one fatty acid residue in one molecule as a result of enzymo lysis.

Furthermore, as glycerophospholipid typified by the above lecithin, hydrogenated or hydroxylated lecithins can also be used in the present invention. For example, hydrogenated lecithins, enzymatically decomposed lecithins, enzymatically decomposed hydrogenated lecithins, and hydroxylecithins can be used.

The hydrogenation is performed by, for example, reacting lecithin with hydrogen in the presence of a catalyst, whereby an unsaturated bond in the fatty acid portion is hydrogenated. The oxidation stability of lecithin is improved by hydrogenation.

With respect to the hydroxylation, an unsaturated bond in the fatty acid portion is hydroxylated by heating lecithin with a high concentration of hydrogen peroxide and an organic acid such as acetic acid, tartaric acid, or butyric acid. The hydrophilicity of lecithin is improved by the hydroxylation.

As the phospholipid, lecithin is particularly preferable in consideration of emulsion stability.

As commercially-available lecithins, LECION series and LECIMAL EL (trade names, manufactured by Riken Vitamin Co., Ltd.) can be used.

In the present invention, products having a purity of lecithin of 80 mass% or higher, which are referred to as "high purity lecithin", are preferable, and products having a purity of lecithin of 90 mass% or higher are more preferable.

In the invention, the phospholipid may be used singly, or a mixture of two or more phospholipids may be used.

In consideration of emulsion stability, the content of phospholipids in the first emulsion may be from 0.01 mass% to 70 mass%, and preferably from 2 mass% to 35 mass%, with respect to the total amount of the first emulsion. In consideration of the emulsion stability and the particle diameter of the second oil droplet particles, the content of phospholipids in the second emulsion is preferably from 0.001 mass% to 20 mass%, and more preferably from 0.002 to 10 mass%, with respect to the total amount of the second emulsion.

### Surfactants

Surfactants which can be used in the invention are not particularly limited as long as they are water soluble surfactants which dissolve in an aqueous solvent. For example, nonionic surfactants whose HLB is 10 or higher, and preferably 12 or higher, are preferable. When the HLB is lower than 10, the emulsification ability may become insufficient. In consideration of emulsion stability, the HLB is preferably 16 or lower.

Surfactants that can be used in the invention are not particularly limited and any of cationic, anionic, amphoteric and nonionic surfactants can be used. In consideration of emulsion stability, nonionic surfactants are preferred. Examples of the nonionic surfactants include glycerin fatty acid esters, organic acid monoglycerides, polyglycerine fatty acid esters, propylene glycol fatty acid esters, polyglycerin condensed ricinoleic-acid esters, sorbitan fatty acid esters, and sucrose fatty acid esters. Among these, polyglycerin fatty acid esters, sorbitan fatty acid esters, and sucrose fatty acid esters are more preferable. The surfactants are not necessarily highly purified products obtained by distillation or the like, and may be reaction mixtures.

The polyglycerin fatty acid ester used in the invention is an ester of a polyglycerin having an average degree of polymerization of 2 or higher (preferably from 6 to 15, and more preferably from 8 to 10) and a C₈₋₁₈ fatty acid, such as caprylic acid, capric acid, lauric acid, myristic acid, palmitic acid, stearic acid, oleic acid, or linoleic acid. Preferable examples of the polyglycerin fatty acid ester include hexaglycerin monooleate, hexaglycerin monostearate, hexaglycerin monopalmitate, hexaglycerin monomyristate, hexaglycerin monolaurate, decaglycerin monooleate, decaglycerin monostearate, decaglycerin monopalmitate, decaglycerin monomyristate, and decaglycerin monolaurate. The polyglycerin fatty acid ester may be used singly, or a mixture of two or more thereof may be used.

In the sorbitan fatty acid ester used in the invention, the fatty acid preferably has 8 or more carbon atoms, and more preferably 12 or more carbon atoms. Preferable examples of the sorbitan fatty acid ester include sorbitan monocaprylate, sorbitan monolaurate, sorbitan monostearate, sorbitan sesquistearate, sorbitan tristearate, sorbitan isostearate, sorbitan sesquiisostearate, sorbitan oleate, sorbitan sesquioleate, and sorbitan trioleate.

In the sucrose fatty acid ester used in the invention, the fatty acid preferably has 12 or more carbon atoms, and more preferably has 12 to 20 carbon atoms. Preferable examples of the sucrose fatty acid ester include sucrose dioleate, sucrose distearate, sucrose dipalmitate, sucrose dimyristate, sucrose dilaurate, sucrose monooleate, sucrose monostearate, sucrose monopalmitate, sucrose monomyristate, and sucrose monolaurate. In the invention, the sucrose fatty acid ester may be used singly, or a mixture of two or more thereof may be used.

In consideration of emulsion stability, the addition amount of the surfactant in the first emulsion is preferably from 0.01 mass% to 70 mass%, and more preferably 0.1 from mass% to 35 mass%, with respect to the total amount of the first emulsion. On the other hand, in consideration of the particle diameters of the second oil droplet particles, the addition amount of the surfactant in the second emulsion is preferably from 0.1 mass% to 50 mass%, more preferably from 0.5 mass% to 25 mass%, and still more preferably from 1 mass% to 20 mass%, with respect to the total amount of the second emulsion.

### (d) Production method

Methods of producing the emulsion composition of the invention are not limited as long as the resulting emulsion composition contains the first oil droplet particles and the second oil droplet particles. Methods including a step of mixing the first emulsion and the second emulsion are preferable.

Both the first emulsion and the second emulsion can be obtained by conventional methods. For example, each of the first emulsion and the second emulsion can be produced by the following steps: (a) dissolving a surfactant (emulsifier) in an aqueous medium (such as water or the like) to obtain a water phase; (b) mixing and dissolving the above-described components (carotenoids, tocopherols, phospholipids or the like) and, as required, other oil-soluble components to obtain an oil phase; and (c) mixing the water phase and the oil phase under stirring to carry out dispersing emulsification, thereby obtaining an emulsion composition.

Any generally-known emulsification methods can be used, such as a natural emulsification method, an interfacial chemical emulsification method, an electrical emulsification method, a capillary emulsification method, a mechanical emulsification method or an ultrasonic emulsification method.

The ratio (by mass) of the oil phase to the water phase in the dispersing emulsification is not particularly limited. The oil phase/water phase ratio (% by mass) is preferably from 0.1/99.9 to 50/50, more preferably from 0.5/99.5 to 30/70, and further preferably from 1/99 to 20/80.

When the oil phase/water phase ratio is set at 0.1/99.9 or higher, since the amount of an effective component is not too low, problems do not tend to occur upon practical use of the emulsion composition, which is preferable. Further, when the oil phase/water phase ratio is set at 50/50 or lower, since the concentration of the surfactant is not too low, emulsion stability of the emulsion composition does not tend to deteriorate, which is preferable.

When the first emulsion is produced, the dispersing emulsification may involve a one-step emulsification operation, or may involve emulsification operation having two or more steps in order to obtain more uniform emulsified particles.

Specifically, it is particularly preferable to use two or more kinds of emulsification device in combination; for example, emulsification with a high-pressure homogenizer or the like may be conducted in addition to a one-step emulsification operation with a general emulsification apparatus (for example, stirrer, impeller stirring, homomixer, or continuous-flow shearing apparatus) utilizing shear action. When a high-pressure homogenizer is used, the liquid droplets in the emulsion may become more uniform. Furthermore, dispersing emulsification may be conducted plural times for the purpose of making the particle sizes of the liquid droplets more uniform.

The temperature condition at dispersing emulsification in the invention is not particularly limited. The temperature is preferably from 10 to 100°C in consideration of stability of the functional oil component. A preferred temperature range may be appropriately selected in accordance with, for example, the melting point of the functional oil component to be used.

Examples of the high-pressure homogenizer include a chamber high-pressure homogenizer having a chamber in which a flow path for the liquid to be treated is fixed and a homogenizing-valve high-pressure homogenizer having a homogenizing valve. Among them, a homogenizing valve high-pressure homogenizer is preferable for the process for producing the emulsion composition of the invention since the width of the flow path of the liquid to be treated can be controlled easily, and the pressure and the flow rate during operation can be set arbitrarily, which broadens the operation range.

Further, although the degree of freedom for operation is low, the chamber high-pressure homogenizer can also be used suitably when a super high-pressure is required; this is because a mechanism for increasing the pressure can be prepared easily.

Examples of the chamber high-pressure homogenizer include MICROFLUIDIZER (trade name, manufactured by Microfluidics), NANOMIZER (trade name, manufactured by Yoshida Kikai Co., Ltd.), and ALTIMIZER (trade name, manufactured by Sugino Machine Limited).

Examples of the homogenizing valve high-pressure homogenizer include Gaulin homogenizer (manufactured by APV), Ranie homogenizer (manufactured by Ranie), high-pressure homogenizer (manufactured by Niro Soavi), homogenizer (manufactured by Sanwa Machine Co., Ltd.), high-pressure homogenizer (manufactured by Izumi Food Machinery Co., Ltd.) and ultrahigh-pressure homogenizer (manufactured by IKA).

In the invention, the processing pressure in the high-pressure homogenizer is preferably 50 MPa or higher, more preferably from 50 to 250 MPa, and further preferably from 100 to 250 MPa.

Further, in order to maintain the particle diameter of the dispersed particles, the emulsion liquid - an emulsified and dispersed composition - is preferably cooled through a cooler within 30 sec, preferably within 3 sec, from passing the chamber.

When the second emulsion is produced in the invention, an interfacial chemical emulsification method such as a PIT emulsification method or a gel emulsification method may be used for forming fine particle emulsion. This method is advantageous in that the energy consumption is small, and is therefore suitable for fine emulsification of a material that easily deteriorates under heat.

An example of generally-used emulsification methods is a method of using mechanical force, that is, a method of applying strong shear force from outside so as to split oil droplets. The most common form of the mechanical force is a high speed, high shear force stirring machine, which may be selected from commercially available devices called homomixers, disper mixers and ultramixers.

Other useful mechanical emulsification apparatuses for reducing the particle size include various commercially-available high-pressure homogenizers. The high-pressure homogenizers can apply large shearing force as compared with a stirring type apparatuses, and reduction in the particle size is possible even when the amount of emulsifier is relatively small.

The high-pressure homogenizers are roughly classified into chamber high-pressure homogenizers having a fixed throttle part, and homogenizing valve high-pressure homogenizers that allow control of throttle opening. Specific examples of the chamber high-pressure homogenizers and homogenizing valve high-pressure homogenizers include the above-described homogenizers usable in the production method of the first emulsion.

Ultrasonic homogenizers are emulsification apparatuses having a simple structure, which are dispersion apparatuses with relatively high energy efficiency. Examples of a high-power ultrasonic homogenizer that can produce the emulsion include Ultrasonic homogenizers US-600, US-1200T, RUS-1200T and MUS-1200T (all trade names, manufactured by Nissei Corporation), and Ultrasonic processors UIP2000, UIP-4000, UIP-8000 and UIP-16000 (all trade names, manufactured by Hielscher). These high-power ultrasonic irradiation apparatuses may be used at a frequency of 25 kHz or less, and preferably from 15 kHz to 20 kHz.

Other known emulsification means include methods of using a static mixer, a microchannel, a micromixer, a membrane emulsification apparatus or the like, each of which requires only a small energy and does not have a stirring portion connected to the outside. These methods are also useful.

The dispersing emulsification may involve a one-step emulsification operation. However, it is preferable to perform emulsification operation having two or more steps in terms of obtaining uniform and fine emulsified particles.

Each of the first emulsion and the second emulsion, respectively containing oil droplet particles with desired particle diameters, can be obtained by controlling factors such as: the HLB, solubility in oil phase, and solubility in water phase of a surfactant containing a phospholipid, the emulsification temperature, and the stirring conditions, in addition to controlling the oil/water phase ratio at dispersing emulsification.

By mixing the first emulsion and the second emulsion thus obtained, the emulsion composition in the invention can be obtained. It is preferable to mix the second emulsion in with the water phase of the first emulsion in consideration of maintenance of the particle diameters and dispersibility of the oil droplet particles in each of the first and second emulsions. The mixing ratio may vary depending on which function of the emulsion is focused on. The mixing ratio of the first emulsion/the second emulsion by mass is preferably in a range of from 50,000/1 to 1/50,000, and more preferably in a range of from 100/1 to 1/10 in consideration of stability of the mixed emulsion.

### (e) Other ingredients

In addition to the above-mentioned ingredients, the emulsion composition of the invention may include, as required, other ingredients such as water, a polyhydric alcohol, a water-soluble compound, a water-soluble polymer compound, an antiseptic agent, an antioxidant, a metal ion chelating agent, or a fragrant material, which are generally used in cosmetic compositions and the like.

### Polyhydric alcohol

In the invention, the polyhydric alcohol has a moisturizing function, a viscosity regulating function and the like. Furthermore, the polyhydric alcohol has the function to decrease interfacial tension between water and the oil or fat components to facilitate the interface expansion, thereby making it easy to form fine and stable particles.

The polyhydric alcohol that can be used in the invention in not particularly limited. Examples of the polyhydric alcohol include glycerin, diglycerin, triglycerin, polyglycerin, 3-methyl-1,3-butanediol, 1,3-butyleneglycol, isopreneglycol, polyethyleneglycol, 1,2-pentanediol, 1,2-hexanediol, propyleneglycol, dipropyleneglycol, polypropyleneglycol, ethyleneglycol, diethyleneglycol, pentaerythritol, neopentylglycol, maltitol, reduced starch syrup, sucrose, lactitol, palatinit, erythritol, sorbitol, mannitol, xylitol, xylose, glucose, lactose, mannose, maltose, galactose, fructose, inositol, pentaerythritol, maltotriose, sorbitan, trehalose, starch-decomposed sugar, and sugar alcohol obtained by reduction of starch-decomposed sugar. The polyhydric alcohol can be used singly, or a mixture of two or more thereof may be used.

### Water-soluble compound

It is preferable to add a water-soluble compound to the emulsion composition of the invention as required, in consideration of a function thereof.

Examples of the water-soluble compound include a water-soluble whitening agent (such as a melanin-pigment inhibiting agent, a melanin-pigment reducing agent, or a melanin-pigment elimination promoting agent), an anti-wrinkle agent (such as a cell activating agent, a production promoting agent for a matrix (e.g., collagen), or a matrix degradation inhibiting agent), an antioxidant (such as vitamin C, a derivative of vitamin C, or a polyphenol), a skin-roughness improving agent (such as a low-molecular moisturizer (e.g., urea, a lactic acid or a pyrrolidonecarboxylic acid), an antiinflammatory agent, or an immunosuppressive agent), a protective agent for stratum corneum (a differentiation promoting agent for stratum corneum, a lipid production promoting agent for stratum corneum, or a lipid degradation inhibiting agent for stratum corneum), an agent for acne treatment, and an astringent. Specific examples thereof include substances described in Hachiro Tagami et al. "Keshohin-Kagaku Guidebook" (published by Fragrance Journal, 2007), pp. 227 to 235.

### Water-soluble polymer compound

The emulsion composition of the invention may contain a water-soluble polymer compound in consideration of controlling viscosity and the like. In particular, saccharides, proteins, and glycoprotein complexes are preferable.

Examples of saccharides include, but are not limited to, monosaccharides, disaccharides, oligosaccharides, polysaccharides, dextrin, starch derivatives, gums, mucopolysaccharides, and celluloses.

Among the above, typical examples include, but are not limited to, agarose, arabinose, amylose, amylopectin, acacia gum, gum arabic, arabinogalactan, alkyl glycoside, alginic acid, sodium alginate, propylene glycol alginate, aldose, inulin, oligosaccharide, ghatti gum, curdlan, carrageenan, galactomannan, galactose, xanthan gum, xylose, xyloglucan, chitin, chitosan, guar gum, cluster dextrin, β-glucan, glucuronic acid, glycogen, glycosaminoglycan, glyceraldehyde, glucosamine, glucose, glucomannan, ketose, chondroitin sulfate, psyllium seed gum, gellan gum, cyclodextrin, sucrose, hydroxyethylcellulose, hydroxypropylcellulose, carboxymethylcellulose, methylcellulose, cellobiose, sorbitol, deoxyribose, dextrin, invert sugar, starch, soybean polysaccharide, sugar alcohol, glycoprotein, tragacanth gum, trehalose, hyaluronic acid, fucose, fructose, pullulan, pectin, heparin, hemicellulose, maltose, mannitol, mannan, lactose, and ribose.

Among these saccharides, gums and polysaccharides are preferable in consideration of dispersion stability due to increase in viscosity, and xanthan gum, gum arabic, and pullulan are more preferable in consideration of the stability of carotenoids.

As the proteins, any polymer or oligomer in which amino acid residues are polymerized through peptide bonds may be used. More preferable are proteins which are naturally-derived and are water soluble.

Proteins may be classified into simple proteins each composed of amino acids and complex proteins each containing a constituent other than amino acid, and both of them are usable. Examples of the simple proteins include gelatin, collagen, casein, fibroin, sericin, keratin, and protamine. Examples of the complex proteins include a glycoprotein which is a protein bonded to a carbohydrate, a lipoprotein which is a protein bonded to a lipid, a metalloprotein which is a protein bonded to a metal ion, a nucleoprotein which is a protein bonded to a ribonucleic acid, and a phosphoprotein which is a protein bonded to a phosphoric acid group.

In general, proteins are often termed based on their raw materials. For example, animal muscle proteins, milk proteins, egg proteins, fishskin proteins, rice proteins, wheat proteins (wheat gluten), soybean proteins, yeast proteins, and bacteria proteins may be mentioned.

In an embodiment, a mixture of two or more of these proteins may be used.

### Amino acids or derivatives thereof

It is preferable for the emulsion composition of the invention to contain an amino acid or a derivative thereof as another functional component.

It is also preferable for the emulsion composition of the invention to contain a dipiptide, a tripeptide or a tetrapeptide in which plural units selected from an amino acid or a derivative thereof are connected.

Usable amino acids or derivatives thereof may be selected from those usable as ingredients of cosmetic materials, without particular limitations.

Examples of the amino acids or derivatives thereof include amino acids such as glycine, alanine, valine, leucine, isoleucine, serine, threonine, aspartic acid, glutamic acid, cystine, methionine, lysine, hydroxylysine, arginine, histidine, phenylalanine, tyrosin, tryptophan, proline, hydroxyproline, and acetylhydroxyproline, and derivatives of such amino acids.

As the amino acids or derivatives thereof, hydroxyproline and acetylhydroxyproline are preferable among the above.

As the amino acids or derivatives thereof, those synthesized by conventional methods and those commercially available are both usable.

The amino acids and derivatives thereof may be used singly or two or more thereof may be used in combination.

### UV absorber

The emulsion composition of the invention may contain a UV absorber. The UV absorber that can be used in the invention may be a known UV absorber, and examples thereof include: benzophenone UV absorbers such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, and 2-hydroxy-4-methoxy-4'-methylbenzophenone; 3-(4'-methylbenzylidene)-d,1-camphor; 3-benzylidene-d,1-camphor; and 4-methoxy-4'-t-butyldibenzoylmethane.

The UV absorber may be contained anywhere in the emulsion composition. However, it is preferable that the UV absorber be contained in the first emulsion in consideration of dispersion stability of the UV absorber. The content of the UV absorber in the emulsion is not limited, and may be generally from 0.1 mass% to 45 mass%, and preferably from 0.5 mass% to 20 mass%, with respect to the total amount of the emulsion composition.

### Fragrant material

The emulsion composition of the invention may contain any fragrant material, for example an animal, vegetable, or mineral-derived natural fragrant material or a synthetic material. Examples of fragrant materials usable in the invention include rose extract, chamomile extract, green tee perfume, lavender oil, geranium oil, jasmine oil, bergamot oil, musk oil, ylang ylang oil, limonene, linalool, β-phenylethyl alcohol, 2,6-nonadienal, citral, cyclopentadecanone, eugenol, rose oxide, indole, phenylacetaldehyde dimethyl acetal, and auranthiol.

The content of the fragrant material is not limited, and may be suitably set. The manner in which the fragrant material is contained in the emulsion composition is not particularly limited. The fragrant material may be contained in either of the first emulsion or the second emulsion, or may be dissolved in the emulsion composition rather than contained in the emulsified particles.

### (f) Physical properties of emulsion composition

The emulsion composition of the invention may be formed into a water dispersion such as face lotion. However, in order to effectively exhibit functions of the functional oil component, the emulsion composition of the invention is preferably in the form of cream or gel.

When the emulsion composition of the invention is in the form of a cream emulsion composition, it is preferable that the content of the oil phase is from 1 mass% to 60 mass%, and more preferably from 5 mass% to 30 mass% with respect to the total amount of the emulsion composition.

When the emulsion composition of the invention is in the form of a cream or gel emulsion composition, the emulsion composition preferably has an appropriate viscosity in consideration of suitability for application to the skin. For example, the viscosity at 25°C is preferably from 200 mPa·s to 200,000 mPa·s, and more preferably from 2,000 mPa·s to 40,000 mPa·s. The viscosity of the cream or gel emulsion composition can be defined as a value measured with a rotational viscometer or a rheometer, and can be measured using a commercially-available viscometer.

The skin external preparation of the invention can effectively exert functions of the functional oil component particularly when applied to the skin, and thus is useful particularly as a cosmetic composition or the like.

### EXAMPLES

Hereinafter, the invention will be described with reference to Examples. The Examples should not be construed as limiting the invention. In the following description, "part" and "%" are based on mass unless otherwise specified.

### Example 1

### (1) Production of emulsion EM-1

The water phase components shown in Table 1 were dissolved under heating at 83°C for 1 hour, and, separately, the oil phase components shown in Table 1 were dissolved under heating at 83°C for 1 hour.

While stirring the oil phase components at 80°C, the water phase components were added thereto. The mixture was emulsified by stirring at 12,000 rpm for 10 minutes by a homomixer. Thereafter, the resultant emulsion was gradually cooled at a rate of 2°C/min while slowly stirred, and the stirring was stopped when the temperature reached 35°C. The resultant emulsion was then allowed to cool, to thereby obtain cream emulsion EM-1.

The particle diameter of emulsion EM-1 was measured by using a dynamic light scattering particle-size-distribution measurement apparatus LB-550 (trade name, manufactured by Horiba, Ltd.). The standard deviation of the volume-particle size distribution was determined from the volume average particle diameter and the volume-particle size distribution obtained by the above measurement. The minimum particle diameter was calculated from the formula: "volume average particle diameter - standard deviation of volume-particle size distribution × 3". The obtained minimum particle diameter was 240 nm.

**Table 1**

| Oil phase | |
|---|---|
| Decaglyceryl monolaurate | 1.000 g |
| Glyceryl monostearate POE (15) | 1.000 g |
| Hydrogenated soybean phospholipid | 1.000 g |
| Stearic acid | 4.000 g |
| Cetanol | 2.000g |
| Behenyl alcohol | 2.000 g |
| Paraffin | 3.000 g |
| Squalane | 12.000 g |
| Jojoba oil | 4.000 g |
| Methyopolysiloxane | 0.200 g |
| Antiseptic agent | Suitable amount |

| Water phase | |
|---|---|
| 1,3-butanediol | 7.000 g |
| L-arginine | 0.100 g |
| Xanthan gum (2% aqueous solution) | 5.000 g |
| Purified water | Balance |
| Total | 100 g |

### (2) Production of emulsion EM-2

Emulsion EM-2 was obtained in the same manner as the production of emulsion EM-1, except that 0.06 g of astaxanthin oil (pigment of Haematococcus alga extracted with supercritical carbon dioxide gas (containing 20 mass% of astaxanthins)) was further added to the oil phase of emulsion EM-1.

The minimum particle diameter of emulsion EM-2 was measured in the same manner as described above, and the obtained minimum particle diameter was 220 nm.

### (3) Production of emulsion EM-3

The water phase components shown below in Table 2 were dissolved under heating at 70°C for 1 hour, and, separately, the oil phase components shown in Table 2 were dissolved under heating at 70°C for 1 hour. The water phase components were stirred at 10,000 rpm by a homogenizer (Vacuum emulsifier PVQ-1D model, manufactured by MIZUHO Industrial CO., LTD.) while the temperature of the water phase components was maintained at 70°C. The oil phase components were then added thereto, to thereby obtain an emulsion. The obtained emulsion was emulsified under a high pressure of 200 MPa at 40°C using an Altimizer HJP-25005 (trade name, manufactured by Sugino Machine Limited).

Thereafter, the resultant emulsion was filtered through a microfilter having an average pore size of 1 µm to thereby obtain astaxanthin-containing emulsion AE-1 (containing 0.2 g of astaxanthins).

99.0 g of purified water and 1.0 g of astaxanthin-containing emulsion AE-1 were mixed and stirred with a stirrer for 10 minutes. The particle diameter of the obtained water-diluted product was measured at 25°C using a dynamic light scattering particle-size-distribution measurement apparatus LB-550 (trade name, manufactured by Horiba, Ltd.), and the obtained average particle diameter was 150 nm.

Emulsion EM-3 (containing 0.06 g of astaxanthins) was obtained in the same manner as the production of emulsion EM-1, except that the amount of purified water was adjusted such that the total amount of EM-1 became 70.0 g and, after the oil phase components and the water phase components of EM-1 were mixed to make the total volume 70.0 g and the mixture was cooled to 35°C, 30 g of astaxanthin-containing emulsion AE-1 (having an average particle diameter of 150 nm) was further added to the water phase of EM-1.

**Table 2**

| Oil phase | |
|---|---|
| Astaxanthin oil (astaxanthins content: 20 mass%) | 1.0 g |
| Mixed tocopherols | 0.25 g |
| Glyceryl tri(caprylate /caprate) | 1.5 g |
| Lecithin | 1.0g |

| Water phase | |
|---|---|
| Sucrose laurate | 0.75 g |
| Polyglyceryl-10 laurate | 0.75 g |
| Purified water | Balance |
| Total | 100 g |

### (3) Production of emulsion EM-4

The water phase shown below in Table 3 were dissolved under heating at 70°C for 1 hour, and, separately, the oil phase components shown in Table 3 were dissolved under heating at 70°C for 1 hour. The water phase components were stirred at 10,000 rpm by a homogenizer (Vacuum emulsifier PVQ-1D model, manufactured by MIZUHO Industrial CO., LTD.) while the temperature of the water phase components was maintained at 70°C. The oil phase components were then added thereto, to thereby obtain an emulsion. The obtained emulsion was emulsified under a high pressure of 200 MPa at 40°C using an Altimizer HJP-25005 (trade name, manufactured by Sugino Machine Limited).

Then, in a manner similar to the preparation of astaxanthin-containing emulsion AE-1, astaxanthin-containing emulsion AE-2 (containing 0.2 g of astaxanthins and having an average particle diameter of 60 nm) was obtained.

Emulsion EM-4 was obtained in the same manner as the production of emulsion EM-3, except that astaxanthin-containing emulsion AE-2 obtained above was added in place of AE-1.

**Table 3**

| Oil phase | |
|---|---|
| Astaxanthin oil (astaxanthins content: 20 mass%) | 1.0 g |
| Mixed tocopherols | 0.25 g |
| Triglyceryl caprylate/caprate | 2.0 g |
| Lecithin | 1.0g |

| Water phase | |
|---|---|
| Sucrose laurate | 0.5 g |
| Polyglyceryl-10 laurate | 0.5 g |
| Purified water | Balance |
| Total | 100 g |

### (5) Production of emulsion EM-5

Astaxanthin-containing emulsion AE-3 (containing 0.2 g of astaxanthins and having an average particle diameter of 150nm) was obtained in the same manner as the production of astaxanthin-containing emulsion AE-1, except that the water phase components and the oil phase components shown in Table 4 were used.

An emulsion BM-5 having a minimum particle diameter of 240 nm was obtained in the same manner as the preparation of emulsion EM-1, except that 0.003 g of astaxanthin oil was further added to the oil phase and the amount of purified water was adjusted such that the total amount became 85 g. Emulsion EM-5 was obtained in the same manner as the production of emulsion EM-3, except that 15 g of astaxanthin-containing emulsion AE-3 was added to the water phase of emulsion BM-5 in place of adding AE-1 to the water phase of EM-1.

**Table 4**

| Oil phase | |
|---|---|
| Astaxanthin oil (astaxanthins content of 20 mass%) | 1.0 g |
| Mixed tocopherols | 0.25 g |
| Triglyceryl caprylate/caprate | 1.0 g |
| Lecithin | 1.0g |

| Water phase | |
|---|---|
| Sucrose laurate | 1.0g |
| Polyglyceryl-10 laurate | 1.0g |
| Purified water | Balance |
| Total | 100 g |

### (6) Production of emulsion EM-6

Emulsion EM-6 was obtained in the same manner as the production of emulsion EM-5, except that 15 g of astaxanthin-containing emulsion AE-2 (containing 0.2 g of astaxanthins and having an average particle diameter of 60 nm) was added to the water phase of emulsion BM-5 in place of adding AE-3 to the water phase of EM-5.

### (7) Production of emulsion EM-7

Emulsion EM-7 (containing 0.06 g of astaxanthins) was obtained in the same manner as the production of emulsion EM-1, except that (i) the amount of purified water was adjusted such that the total amount of EM-1 became 70 g and (ii) 15 g of astaxanthin-containing emulsion AE-1 (containing 0.2 g of astaxanthins and having an average particle diameter of 150 nm) and 15 g of astaxanthin-containing emulsion AE-2 (containing 0.2 g of astaxanthins and having an average particle diameter of 60 nm) were further added to 70 g of the water phase of emulsion EM-1, followed by stirring.

### (8) Evaluation method

Emulsions EM-1 to 7 were evaluated as follows. The test subjects were 5 women aged from 35 to 55 years. Each test subject applied two types of emulsions to each half of the face twice a day (in the morning and in the evening). EM-1 was applied to half of the face and EM-2 was applied to the other half of the face. This half face test was repeated for all of emulsions EM-1 to 7 by replacing EM-2 with EM-3 to 7. The sense of use of each of emulsions EM-1 to EM-7 (improvements in moist feeling, soft feeling and plump feeling of the skin) was evaluated on the first day and the fifth day of use based on the following criteria.
5 points: Sensed strongly improved effects
4 points: Sensed improved effects
3 points: Sensed no effect
2 points: Sensed adverse effects
1 point: Sensed strongly adverse effects.

The average of the points given by the five women was calculated and used for evaluation.

It should be noted that the "moist feeling of the skin" is evaluation of the moisture-retaining property of the stratum corneum, the "soft feeling of the skin" is evaluation of the skin flexibility, and the "plump feeling of the skin" is evaluation of the moisture-retaining property of a skin layer below the stratum corneum.

The results for the respective emulsions are shown in Table 5. It should be noted that the particle diameter of the astaxanthin (AX)-containing oil droplet particles shown in Table 5 indicates (i) the minimum particle diameter if the particles were obtained by addition to the oil phase of the emulsion or (ii) the average particle diameter if the particles were obtained by addition to the water phase of the emulsion (i.e., when the astaxanthin oil was added for forming a preliminary emulsion such as AE-1, 2 or 3).

**Table 5**

| Sample | Particle diameter of AX-containing oil droplet particle (nm) Oil phase/Water phase | Improvement in moist feeling of the skin | | Improvement in soft feeling of the skin | | Improvement in plump feeling of the skin | |
|---|---|---|---|---|---|---|---|
| | | 1st day | 5th day | 1st day | 5th day | 1st day | 5th day |
| EM-1 | -/- | 3.8 | 3.8 | 3.6 | 3.8 | 3.4 | 3.6 |
| | | ±0 | | +0.2 | | +0.2 | |
| EM-2 | 220/- | 3.8 | 3.8 | 3.6 | 4.0 | 3.4 | 3.8 |
| | | ±0 | | +0.4 | | +0.4 | |
| EM-3 | -/150 | 3.8 | 3.8 | 3.6 | 4.0 | 3.6 | 4.0 |
| | | ±0 | | +0.4 | | +0.4 | |
| EM-4 | -/60 | 4.0 | 4.2 | 3.8 | 4.2 | 3.6 | 4.2 |
| | | +0.2 | | +0.4 | | +0.6 | |
| EM-5 | 240/150 | 4.0 | 4.0 | 3.6 | 4.0 | 3.6 | 4.0 |
| | | ±0 | | +0.4 | | +0.4 | |
| EM-6 | 240/60 | 4.0 | 4.2 | 3.8 | 4.4 | 3.6 | 4.4 |
| | | +0.2 | | +0.6 | | +0.8 | |
| EM-7 | -/150,60 | 3.8 | 4.0 | 3.8 | 4.0 | 3.8 | 4.0 |
| | | +0.2 | | +0.2 | | +0.2 | |

As shown in Table 5, it was found that emulsion EM-6, which contained two kinds of oil droplet particles each containing astaxanthins (i.e., the oil droplet particles having a minimum particle diameter of 240 nm or more and the oil droplet particles having an average particle diameter of 70 nm or less), received high evaluation even on the first day. In addition, it was found that, when emulsion EM-6 was used, the sense of use on the 5th day was remarkably improved in terms of the moist feeling, soft feeling and plump feeling of the skin, as compared with other emulsions.

When emulsion EM-6 was used, the sense of use on the 5th day was improved also in terms of the resilient feeling of skin as compared with other emulsions.

Thus, it was found that emulsion EM-6 of the invention could improve the functions of astaxanthins relating to skin flexibility.

Therefore, the functions of the functional oil component can be improved according to the present invention.

## Claims

1. A skin external preparation comprising an oil-in-water emulsion composition, in which the emulsion composition contains at least first oil droplet particles having a minimum particle diameter of 200 nm or more and second oil droplet particles having a volume average particle diameter determined by a dynamic light scattering method of 70 nm or less, the first oil droplet particles include a first functional oil component, and the second oil droplet particles include a second functional oil component which may be the same as or different from the first functional oil component wherein the minimum particle diameter refers to a value obtained by subtracting a value that is three times the standard deviation of a volume-particle size distribution from a volume average particle diameter, the volume-particle size distribution and the volume average particle diameter being determined by a dynamic light scattering method, wherein each of the first functional oil component and the second functional oil component is independently at least one selected from the group consisting of an oil and a fat, a hydrocarbon, a wax, an ester, a fatty acid, a higher alcohol, a polymer, an oil-soluble colorant and an oil-soluble protein, and wherein at least one of the first functional oil component or the second functional oil component comprises at least one of astaxanthin or an ester of astaxanthin.

2. The skin external preparation according to claim 1, wherein at least one of the first functional oil component or the second functional oil component comprises a Haematococcus alga extract.

3. The skin external preparation according to either claim 1 or claim 2, wherein the content of the first oil droplet particles is from 0.005 mass% to 60 mass% with respect to the total amount of the emulsion composition.

4. The skin external preparation according to any one of claims 1 to 3, wherein the content of the second oil droplet particles is from 0.005 mass% to 60 mass% with respect to the total amount of the emulsion composition.

5. The skin external preparation according to any one of claims 1 to 4, wherein the blending ratio of the first oil droplet particles to the second oil droplet particles in the emulsion composition is from 50,000:1 to 1:50,000 by mass.

6. The skin external preparation according to any one of claims 1 to 5, wherein the total blending amount of oil phase components in the oil-in-water emulsion composition is from 0.01 mass% to 60 mass% with respect to the total amount of the emulsion composition.

7. The skin external preparation according to any one of claims 1 to 6, further comprising at least one water-soluble compound selected from the group consisting of a whitening agent, an anti-wrinkle agent, an antioxidant, a skin-roughness improving agent, a protective agent for stratum corneum, an agent for acne treatment and an astringent.

8. A method of producing the skin external preparation according to any one of claims 1 to7 comprising:
mixing a first emulsion containing the first oil droplet particles having a minimum particle diameter of 200 nm or more and a second emulsion containing the second oil droplet particles having an average particle diameter of 70 nm or less.

9. The method of producing the skin external preparation according to claim 8, wherein the mixing ratio of the first emulsion and the second emulsion is from 50,000:1 to 1:50,000 by mass.

10. The method of producing the skin external preparation according to claim 8 or 9, wherein the content of the first functional oil component in the first emulsion is from 5 mass% to 50 mass%, and the content of the second functional oil component in the second emulsion is from 5 mass% to 35mass%.

## Patentansprüche

1. Zubereitung zur äußeren Anwendung auf der Haut umfassend eine Öl-in-Wasser Emulsionszusammensetzung, in der die Emulsionszusammensetzung mindestens erste Öltropfenpartikel, die einen minimalen Partikeldurchmesser von 200 nm oder mehr aufweisen und zweite Öltropfenpartikel enthält, die einen durch ein dynamisches Lichtstreuungsverfahren bestimmten volumendurchschnittlichen Partikeldurchmesser von 70 nm oder weniger aufweisen, wobei die ersten Öltropfenpartikel eine erste funktionale Ölkomponente und die zweiten Öltropfenpartikel eine zweite funktionale Ölkomponente, die gleich oder unterschiedlich zu der ersten funktionalen Ölkomponente sein kann, enthalten, wobei sich der minimale Partikeldurchmesser auf einen Wert bezieht, der durch Subtrahieren eines Wertes, der dem Dreifachen der Standardabweichung einer Volumen-Partikelgrößenverteilung eines volumendurchschnittlichen Partikeldurchmessers entspricht, erhalten wird, wobei die VolumenPartikelgrößenverteilung und der volumendurchschnittliche Partikelgrößendurchmesser durch ein dynamisches Lichtstreuungsverfahren bestimmt werden, wobei jede der ersten funktionalen Ölkomponente und der zweiten funktionalen Ölkomponente unabhängig mindestens eine, ausgewählt aus der Gruppe bestehend aus einem Öl und einem Fett, einem Kohlenwasserstoff, einem Wachs, einem Ester, einer Fettsäure, einem höheren Alkohol, einem Polymer, einem öllöslichen Farbstoff und einem öllöslichen Protein ist, und wobei die erste funktionale Ölkomponente und/oder die zweite funktionale Ölkomponente mindestens eines aus Astaxanthin oder einem Ester von Astaxanthin enthält.

2. Zubereitung zur äußeren Anwendung auf der Haut nach Anspruch 1, wobei die erste funktionale Ölkomponente und/oder die zweite funktionale Ölkomponente Haematococcus Algenextrakt enthält.

3. Zubereitung zur äußeren Anwendung auf der Haut nach entweder Anspruch 1 oder 2, wobei der Gehalt an den ersten Öltropfenpartikeln, bezogen auf die Gesamtmenge der Emulsionszusammensetzung, von 0,005 Masse% bis 60 Masse% beträgt.

4. Zubereitung zur äußeren Anwendung auf der Haut nach einem der Ansprüche 1 bis 3, wobei der Gehalt an den zweiten Öltropfenpartikeln, bezogen auf die Gesamtmenge der Emulsionszusammensetzung, von 0,005 Masse% bis 60 Masse% beträgt.

5. Zubereitung zur äußeren Anwendung auf der Haut nach einem der Ansprüche 1 bis 4, wobei das Mischungsverhältnis der ersten Öltropfenpartikel zu den zweiten Öltropfenpartikeln in der Emulsionszusammensetzung, bezogen auf die Masse, von 50000 : 1 bis 1 : 50000 beträgt.

6. Zubereitung zur äußeren Anwendung auf der Haut nach einem der Ansprüche 1 bis 5, wobei die Gesamtmischungsmenge an Ölphasenkomponenten in der Öl-in-Wasser-Emulsionszusammensetzung, bezogen auf die Gesamtmenge der Emulsionszusammensetzung, von 0,01 Masse% bis 60 Masse% beträgt.

7. Zubereitung zur äußeren Anwendung auf der Haut nach einem der Ansprüche 1 bis 6, ferner enthaltend mindestens eine wasserlösliche Verbindung, ausgewählt aus der Gruppe bestehend aus einem Weißtöner, einem Anti-Falten-Mittel, einem Antioxidans, einem Mittel, das die Hautrauheit verbessert, einem Mittel, das das Stratum Corneum schützt, einem Mittel zur Aknebehandlung und einem Adstringens.

8. Verfahren zur Herstellung der Zubereitung zur äußeren Anwendung auf der Haut nach einem der Ansprüche 1 bis 7, umfassend:
ein Mischen einer ersten Emulsion, die erste Öltropfenpartikel enthält, die einen minimalen Partikeldurchmesser von 200 nm oder mehr aufweisen, und einer zweiten Emulsion, die zweite Öltropfenpartikel enthält, die einen durchschnittlichen Partikeldurchmesser von 70 nm oder weniger aufweisen.

9. Verfahren zur Herstellung der Zubereitung zur äußeren Anwendung auf der Haut nach Anspruch 8, wobei ein Mischungsverhältnis der ersten Emulsion und der zweiten Emulsion, bezogen auf die Masse, von 50000 : 1 bis 1 : 50000 beträgt.

10. Verfahren zur Herstellung der Zubereitung zur äußeren Anwendung auf der Haut nach Anspruch 8 oder 9, wobei der Gehalt an der ersten funktionalen Ölkomponente in der ersten Emulsion von 5 Masse% bis 50 Masse% beträgt, und der Gehalt an der zweiten funktionalen Ölkomponente in der zweiten Emulsion von 5 Masse% bis 35 Masse% beträgt.

## Revendications

1. Préparation externe pour la peau, comprenant une composition d'émulsion huile dans eau, dans laquelle la composition d'émulsion contient au moins des premières particules de gouttelettes d'huile présentant un diamètre de particule minimum supérieur ou égal à 200 nm et des secondes particules de gouttelettes d'huile présentant un diamètre de particule moyen en volume, déterminé par un procédé de diffusion dynamique de la lumière, inférieur ou égal à 70 nm ; les premières particules de gouttelettes d'huile incluent un premier composant huileux fonctionnel et les secondes particules de gouttelettes d'huile incluent un second composant huileux fonctionnel qui peut être identique ou différent du premier composant huileux fonctionnel, dans laquelle le diamètre de particule minimal fait référence à une valeur obtenue en soustrayant une valeur égale à trois fois l'écart type de la répartition granulométrique en volume du diamètre de particule moyen en volume, la répartition granulométrique en volume et le diamètre de particule moyen en volume étant déterminés par un procédé de diffusion dynamique de la lumière, dans laquelle chacun des premier composant huileux fonctionnel et second composant huileux fonctionnel est indépendamment au moins un élément sélectionné parmi le groupe consistant une huile et une graisse, un hydrocarbure, une cire, un ester, un acide gras, un alcool supérieur, un polymère, un colorant soluble dans l'huile, et une protéine soluble dans l'huile, et dans laquelle au moins un des premier composant huileux fonctionnel ou second composant huileux fonctionnel comprend au moins un élément parmi l'astaxanthine ou un ester d'astaxanthine.

2. Préparation externe pour la peau selon la revendication 1, dans laquelle au moins un des premier composant huileux fonctionnel ou second composant huileux fonctionnel comprend un extrait d'algue Haematococcus.

3. Préparation externe pour la peau selon la revendication 1 ou 2, dans laquelle la teneur des premières particules de gouttelettes d'huile s'étend de 0,005 % en masse à 60% en masse par rapport à la quantité totale de la composition d'émulsion.

4. Préparation externe pour la peau selon l'une quelconque des revendications 1 à 3, dans laquelle la teneur des secondes particules de gouttelettes d'huile s'étend de 0,005 % en masse à 60% en masse par rapport à la quantité totale de la composition d'émulsion.

5. Préparation externe pour la peau selon l'une quelconque des revendications 1 à 4, dans laquelle le rapport de mélange entre les premières particules de gouttelettes d'huile et les secondes particules de gouttelettes d'huile dans la composition d'émulsion s'étend de 50 000 :1 à 1 : 50 000 en masse.

6. Préparation externe pour la peau selon l'une quelconque des revendications 1 à 5, dans laquelle la quantité totale de mélange des composants de phase huileuse dans la composition d'émulsion huile dans eau s'étend de 0,01 % en masse à 60 % en masse par rapport à la quantité totale de la composition d'émulsion.

7. Préparation externe pour la peau selon l'une quelconque des revendications 1 à 6, comprenant en outre au moins un composé soluble dans l'eau sélectionné parmi le groupe consistant en un agent de blanchiment, un agent antirides, un antioxydant, un agent d'amélioration de la rugosité de la peau, un agent protecteur pour la couche cornée, un agent de traitement de l'acné, et un astringent.

8. Procédé destiné à produire la préparation externe pour la peau selon l'une quelconque des revendications 1 à 7, comprenant l'étape consistant à :
mélanger une première émulsion contenant les premières particules de gouttelettes d'huile présentant un diamètre de particule minimum supérieur ou égal à 200 nm et une seconde émulsion contenant les secondes particules de gouttelettes d'huile présentant un diamètre de particule moyen en volume inférieur ou égal à 70 nm.

9. Procédé destiné à produire la préparation externe pour la peau selon la revendication 8, dans lequel le rapport de mélange entre la première émulsion et la seconde émulsion s'étend de 50 000 :1 à 1 : 50 000 en masse.

10. Procédé destiné à produire la préparation externe pour la peau selon la revendication 8 ou 9, dans lequel la teneur du premier composant huileux fonctionnel dans la première émulsion s'étend de 5 % en masse à 50% en masse, et la teneur du second composant huileux fonctionnel dans la seconde émulsion s'étend de 5 % en masse à 35% en masse.
